# EUROPEAN PATENT SPECIFICATION

(11) **EP 0 565 522 B1**
(45) Date of publication and mention of the grant of the patent: **07.05.1997**
(21) Application number: 91909326.0
(22) Date of filing: 29.03.1991
(51) Int. Cl.: C12N 1/04, C12N 11/04, C12N 1/20

(54) **DRIED, ROTARY DISC FATTY ACID MICROENCAPSULATED BACTERIA**
GETROCKNETE, AUF DREHSCHEIBEN MIT FETTSÄUREN MIKROEINGEKAPSELTE BAKTERIEN
BACTERIES SECHEES, MICROENCAPSULEES AVEC UN ACIDE GRAS, AU MOYEN D' UN DISQUE ROTATIF

(30) Priority: 31.12.1990 US 635973
(43) Date of publication of application: 20.10.1993
(73) Proprietor: PIONEER HI-BRED INTERNATIONAL, INC., Des Moines Iowa 50309 (US)
(72) Inventor: RUTHERFORD, William, M., Des Moines, IA 50310 (US); ALLEN, Jack, E., Booneville, Iowa 50038 (US); SCHLAMEUS, Herman, Wade, San Antonio, TX 78250 (US); MANGOLD, Donald, J., San Antonio, TX 78248 (US); HARLOWE, William, W., Jr., San Antonio, TX 78230 (US)
(74) Representative: Thomson, Paul Anthony
(86) International application number: US9102118
(87) International publication number: WO9212234

(56) References cited:
- GB-A- 2 016 043
- US-A- 1 260 899
- US-A- 2 369 218
- US-A- 3 856 699
- US-A- 3 959 493
- US-A- 4 332 790
- US-A- 4 352 883
- US-A- 4 386 895
- US-A- 4 675 140
- US-A- 4 692 284
- Catalogue of Bacteria and Bacteriophages, 17th edition, published 1989, by American Type Culture Collection, see page 79.

## Description

### BACKGROUND OF THE INVENTION

It is known that certain bacteria are potentially beneficial when added to animal feeds. These bacteria are beneficial in that they supply a natural intestinal micro-flora. Some companies offer for sale probiotics which contain desirable bacteria. Probiotics, however, do have some difficulty in maintaining a stable product. Typically, the probiotic is used at a fairly small level, added to feed at perhaps a 1% level. However, unused probiotic containing feed or feed additive product is often stored by the farmers for long periods of time. This storage many times is under conditions where there is some moisture. In many instances there is just enough moisture that the bacteria are activated or start to grow, but yet there is an insufficient amount of moisture to sustain them. As a result they die. Thus, the activity of the probiotic is stopped. In other instances, the addition of antibiotics to the probiotic containing feed or feed additive adversely interacts with the bacteria, particularly if there are small amounts of moisture present and thus again bacteria are killed. Thus, there is a significant problem of long term storage stability for probiotics.

In another environment, where the probiotic is added to, for example chicken feed, it is common to pelletize the material with the probiotic added before pelletizing. Moisture from steam used during pelletization partially activates the bacteria, but may, as a result of insufficient moisture to sustain them, kill them. Also heat during pelletization may kill them. Then, too, there is the problem of the acid environment of the stomach potentially inactivating bacteria before they really reach the intestine. Thus, there is a continuing need for probiotics which will release the organisms only at the proper time in the intestine, without early release due to moisture conditions or adverse pH conditions such as exist in the digestive tract anterior to the small intestine.

It is a primary objective of the present invention to provide probiotics suitable for animal feed ration addition which contains bacteria that are microencapsulated in a special rotary encapsulation technique using free fatty acid encapsulant.

Another objective of the present invention is to provide a probiotic which has stability at levels within the range of from 3 months to 6 months without any significant organism count reduction.

Another objective of the present invention is to provide a process of rotary microencapsulation of dried bacteria which provides individual spheres of coated bacteria as opposed to clumped, clusters of spheres of coated bacteria.

Another objective of the present invention is to provide rotary disc microencapsulated dried bacteria which are free flowing, and easily processible with animal feed rations.

An even further objective of the present invention is to provide microencapsulated Enteroococcus faceium.

### BRIEF DESCRIPTION OF THE DRAWINGS

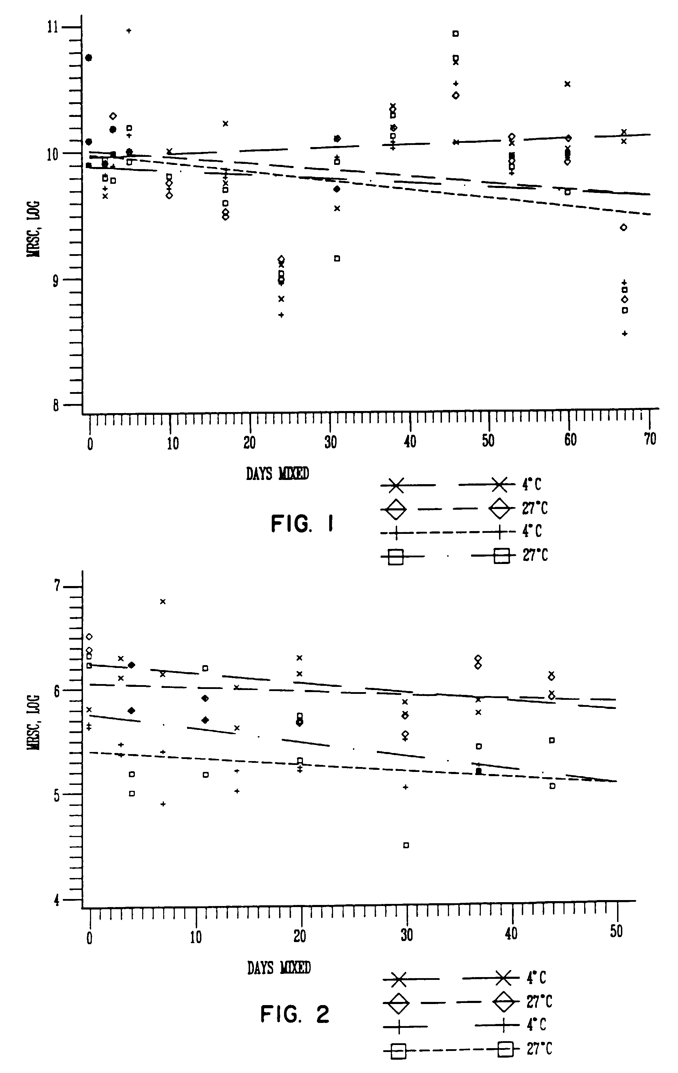

Figures 1, 2 and 3 show graphically the stability of encapsulated strains using stearic acid encapsulant.

According to the present invention there is provided discrete individual capsules of fatty acid microencapsulated bacteria, produced by mixing freeze dried bacterial culture with a fatty acid and rotary disc encapsulating said mix.

Discrete particles of fatty acid microencapsulated bacteria, preferably stearic acid encapsulated Enterococcus faceim are provided. Freeze dried bacterial culture is preferably mixed with from 50% to over 90% by weight of stearic acid melt and thereafter rotary disc encapsulated.

Also in accordance with the present invention there is provided a process of processing discrete individual capsules of fatty acid microencapsulated bacteria, said process comprising:
obtaining a bacterial culture of dried bacteria,
mixing said freeze dried culture with from 50% to over 90% by weight of a fatty acid melt, and
rotary disc encapsulating the mix of fatty acid and freeze dried bacteria in a rotary disc encapsulator to provide individually coated free flowing capsules of bacteria.

### DETAILED DESCRIPTION OF THE INVENTION

This invention relates to freeze dried, rotary disc microencapsulated capsules of bacteria. Preferably the capsules are free fatty acid microencapsulated. There are two significant and important aspects of this invention that distinguish it from other prior encapsulated bacteria. In the first instance it is the nature of the encapsulant, that is a fatty acid. In the second instance it is the nature of the encapsulation process, which here does not use a conventional spray drying technique but instead a method of rotary disc microencapsulation. It is the coaction of these two distinct steps which provide for the highly stable probiotic of the present invention. If either step alone is used, the stability is not achieved.

The preferred encapsulating agent is a C₁₂ to C₂₄ free fatty acid. While mixtures of fatty acids may be employed, it is preferred that a single pure free fatty acid be employed. It is also preferred that the free fatty acid be an unsaturated fatty acid, with the most preferred being stearic acid.

Generally speaking, it is important that the fatty acid have a melting point less than 75°C, preferably within the range of 40°C to 75°C. It must, of course, be solid at room temperature in order to be an effective encapsulant. All free fatty acids falling within the range of chemical description heretofore given will meet these requirements.

The precise bacteria encapsulated is not critical. However, the precise one selected will depend upon the probiotic being formed. Generally speaking, for use in this invention, Enterococcus faceium is the preferred bacteria. It should be understood that other bacteria such as Lactobacillus, Bacillus, etc. may also be employed. Mixtures of strains may be employed as well as individual strains. In order to enhance the product stability, the bacteria are typically freeze-dried bacteria as placed in the product. Thus, they can be revived by moisture addition.

In the microencapsulant, made in accordance with the process discussed below, the microencapsulated particles generally comprise from about 50% to over 90% by weight of the fatty acid component with the balance being bacterial culture. The preferred range is from about 60% to about 75% fatty acid. If too little fatty acid is used, the coating will be inadequate for protection. On the other hand, if too much is used, the coating will be too thick and results in inadequate release in the gut.

The encapsulation process as used in this invention is a rotary disc microencapsulation process. Generally speaking in the rotary disc technology, a slurry of the bacteria and fatty acid components are thoroughly mixed with the mixture being added at a uniform rate onto the center of a rotating stainless steel disc. It is there flung outwardly as a result of centrifugal force. It is then collected in a cooling chamber maintained at ambient conditions or slightly lower, sized and readied for packaging.

While rotary disc encapsulation is known, it is not known for use with bacteria for microencapsulation of freeze dried bacteria. Generally speaking for descriptions of rotary disc encapsulation, see a paper by Johnson, et al. of the Southwest Research Institute of San Antonio, in the Journal of Gas Chromotography, October, 1965, pages 345-347. In addition, a rotary disc encapsulator suitable for use in this invention is described in detail in United States Letters Patent, Sparks, 4,675,140, issued June 23, 1987 and entitled "Method For Coating Particles For Liquid Droplets the disclosure of which is incorporated herein by reference.

It is important to note that rotary microencapsulation provides a distinctly different product than does conventional tower spray drying. In conventional tower spray drying there is a tendency for particles to cluster, for the coating to be uneven, and thus for the stability of the product to be significantly effected perhaps from days to weeks. When rotary microencapsulation, particularly with encapsulating agents used in this invention is used, the stability of the resulting bacteria, even when subjected to some moisture and antibiotics, will be for from three to six months.

When the microencapsulant free fatty acid material of the present invention is used within the ranges hereinbefore expressed, the encapsulator typically employing a 4" rotary disc can be run at the rate of from 2000 rpm to 4000 rpm, preferably about 2500 rpm to 3200 rpm with a feed rate of from 50 grams to 200 grams per minute. The preferred conditions presently known are use of stearic acid, use of Enterococcus faceium, a four inch rotary disc, 3000 rpm and a feed rate of 100 grams per minute with a bacteria/stearic acid slurry of 35% bacteria, 65% stearic acid. When this is done, a product having a particle size of from 75 microns to 300 microns will be achieved, with a preferred level of less than 250 microns.

The following examples are offered to further illustrate, but not limit, the process of the present invention. The examples are described in connection with Figures 1, 2 and 3.

### Example 1

Example 1 correlates with Figure 1. It shows the product stability of two different strains of Enterococcus faceium with temperatures of 4°C and 27°C. As illustrated in Figure 1, it shows a stability of the encapsulated strains of Enterococcus faceium, with the encapsulation being by the rotary disc encapsulator using stearic acid with a level of 35% culture weight. Conditions of encapsulation were as previously described herein, namely a 35/65 bacteria stearic acid slurry at a temperature of 60°C, using a four inch rotary disc, operating at 3000 rpm and a feed rate of 100 grams per minute. The culture was encapsulated, placed in heat sealed vapor barrier pouches and destructively sampled weekly for CFU determination. It can be seen that the product of the invention maintained excellent organism colony forming unit (CFU) counts out to storage times aS long as 70 days.

### Example 2

Example 2 is to be interpreted in connection with Figure 2. The figure shows the stability of individual encapsulated strains when mixed in a typical feed ration in the presence of three poultry antibiotics. The ration consisted of the following:

| | |
|---|---|
| 54% | five cracked corn |
| 26% | soybean meal |
| 2% | fish meal moisture content: 12% |
| 1.5% | dicalcium phosphate |
| 1% | limestone |
| 5.5% | soy oil |

Three antibiotics were added at the following inclusion rates by weight: Deccox 6% (454 ppm), Salinomycin (50 ppm) and monensin sodium (120 ppm).

Culture was added to the mixture at a level to deliver approximately 1x10⁶ CFU/gm feed (100-150 gm/ton). Feed was packaged in heat sealed bags and incubated at room temperature. Samples were taken weekly for CFU determination. The graph of Figure 2 illustrates the excellent stability.

### Example 3

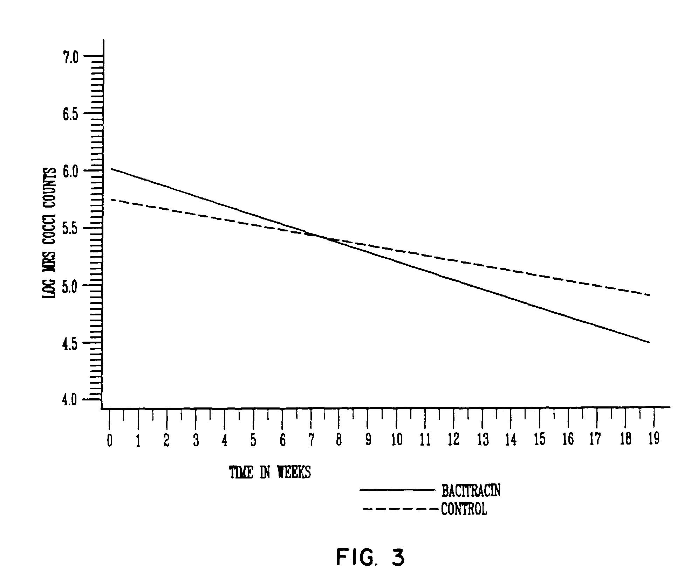

Example 3 is to be interpreted in conjunction with Figure 3. It shows the stability of the encapsulated Enterococcus faceium mixture in feed in the presence of different antibiotics. The ration consisted of 60% fine cracked corn, 38% soybean meal and 2% limestone with a moisture content of about 14%. Culture was added to a level of approximately 10⁶ CFU/gm feed and mixed. Ten pound aliquots were stored in sealed bags at 20 C and sampled weekly for 16 weeks. The antibiotics were included in the ration at the following levels:

| | |
|---|---|
| Bacitracin methylene disalicylate | 50 gm/ton |
| Carbadox | 50 gm/ton |
| Chlortetracycline | 200 gm/ton |
| Lasalocid | 30 gm/ton |
| Lincomycin | 100 gm/ton |
| Neomycin | 140 gm/ton |
| Oxytetracycline | 150 gm/ton |
| Sulfamethazine | 100 gm/ton |
| Tylosin | 100 gm/ton |
| Virginiamycin | 20 gm/ton |
| ASP250 | 100 gm/ton |

Table 1 is a list of the minimum times for a 1 log loss in colony forming units (CFU).

**Table 1**

| Time in days for loss of 1 log CFU counts at 20C in 14% moisture mash feed. | |
|---|---|
| Antibiotic | Time of Storage (days) |
| Control | 103 |
| Bacitracin | 88 |
| Carbadox | 54 |
| Chlortetracycline | 60 |
| Lasalocid | 57 |
| Linocomycin | 75 |
| Neomycin | 53 |
| Oxytetracycline | 59 |
| Sulfamethazine | 62 |
| Tylosin | 52 |
| Virginianycin | 112 |
| ASP250 | 67 |
| Furadox | 53 |

### Example 4

In Example 4 the stability of product after pelletizing for use of a chicken feed product was determined. The microencapsulation conditions were as earlier described. The conditions used in this study were the following:

| | |
|---|---|
| Crude Protein, not less than | 18.0% |
| Crude Fat, not less than | 5.0% |
| Crude Fiber, not more than | 6.0% |

The pellets with and without the antibiotic (CTC 50 gm/ton) were made with the following ingredients and conditions.

Corn, SEM, whey, soy oil, dicalcium phosphate, limestone, trace mineral premix, vitamin premix, selenium, copper sulfate. Culture was added at approximately 5x10⁵ CFU/gm feed (100-150 gm/ton).

Conditioning temperature was 70°C and the pellets out of the dye were 78°C.

Pellets were stored in unsealed bags and sampled weekly for CFU determination.

In each instance the pelletized product was not adversely affected in stability by the conditions of pelletizing. In particular, the pelletized product showed stability equal to the unpelletized product.

It therefore can be seen that the invention accomplishes all of its stated objectives.

## Claims

1. Discrete individual capsules of fatty acid microencapsulated bacteria, producable by mixing freeze dried bacterial culture with a fatty acid and rotary disc encapsulating said mix.

2. The microencapsulated bacteria as claimed in claim 1, characterised in that the fatty acid is a C₁₂ to C₂₄ free fatty acid.

3. The microencapsulated bacteria as claimed in claim 2, characterised in that the fatty acid is stearic acid.

4. The microencapsulated bacteria as claimed in claim 1, 2 or 3, characterised in that the microencapsulated capsules are from 50% to 90% fatty acid, with the balance being bacterial culture.

5. The microencapsulated bacteria as claimed in any preceding claim, characterised in that the capsules have a particle size of from 75 microns to 300 microns.

6. The microencapsulated bacteria as claimed in any preceding claim, characterised in that the bacteria are Enterococcus faceium.

7. A process of processing discrete individual capsules of fatty acid microencapsulated bacteria, said process comprising:
obtaining a bacterial culture of dried bacteria,
mixing said freeze dried culture with from 50% to over 90% by weight of a fatty acid melt, and
rotary disc encapsulating the mix of fatty acid and freeze dried bacteria in a rotary disc encapsulator to provide individually coated free flowing capsules of bacteria.

8. A process as claimed in claim 7, characterised in that the rotary disc encapsulator operates at an rpm within the range of 2000 rpm to 4000 rpm.

9. A process as claimed in claim 7 or 8, characterised in that the rotary disc encapsulator operates at a range of from 2500 rpm to 3200 rpm.

10. A process as claimed in claim 7, 8 or 9, characterised in that the feed rate of material into the rotary disc encapsulator is from 50 grams per minute to 200 grams per minute.

11. A process as claimed in any one of claims 7 to 10, characterised in that the free fatty acid is a C₁₂ to C₂₄ free fatty acid.

12. A process as claimed in claim 11, characterised in that the free fatty acid is stearic acid.

13. A process as claimed in any one of claims 7 to 12, characterised in that the bacteria are Enterococcus faceium.

## Patentansprüche

1. Diskrete Einzelkapseln von mit Fettsäuren mikroeingekapselten Bakterien, herstellbar durch Mischen einer gefriergetrockneten Bakterienkultur mit einer Fettsäure und Einkapseln dieser Mischung auf einer Drehscheibe.

2. Mikroeingekapselte Bakterien nach Anspruch 1, dadurch gekennzeichnet, daß die Fettsäure eine C₁₂ bis C₂₄ freie Fettsäure ist.

3. Mikroeingekapselte Bakterien nach Anspruch 2, dadurch gekennzeichnet, daß die Fettsäure Stearinsäure ist.

4. Mikroeingekapselte Bakterien nach Anspruch 1, 2 oder 3, dadurch gekennzeichnet, daß die durch Mikroeinkapselung hergestellten Kapseln 50 % bis 90 % Fettsäure enthalten, wobei die Restmenge Bakterienkultur ist.

5. Mikroeingekapselte Bakterien nach einem der voranstehenden Ansprüche, dadurch gekennzeichnet, daß die Kapseln eine Teilchengröße von 75 Mikron bis 300 Mikron haben.

6. Mikroeingekapselte Bakterien nach einem der voranstehenden Ansprüche, dadurch gekennzeichnet, daß es sich bei den Bakterien um Enterococcus faceium handelt.

7. Verfahren zur Herstellung diskreter Einzelkapseln von mit Fettsäuren mikroeingekapselten Bakterien, mit folgenden Verfahrensschritten:
Gewinnen einer Bakterienkultur aus getrockneten Bakterien;
Mischen der gefriergetrockneten Kultur mit 50 Gew.-% bis zu über 90 Gew.-% einer Fettsäuremischung; und
Drehscheiben-Einkapseln dieser Mischung aus Fettsäure und gefriergetrockneten Bakterien mittels eines Drehscheiben-Einkapselgeräts zur Herstellung einzeln beschichteter, frei fließender Bakterienkapseln.

8. Verfahren nach Anspruch 7, dadurch gekennzeichnet, daß das Drehscheiben-Einkapselgerät mit einer Drehzahl im Bereich zwischen 2000 und 4000 Umdrehungen pro Minute betrieben wird.

9. Verfahren nach Anspruch 7 oder 8, dadurch gekennzeichnet, daß das Drehscheiben-Einkapselgerät mit einer Drehzahl im Bereich zwischen 2500 und 3200 Umdrehungen pro Minute betrieben wird.

10. Verfahren nach Anspruch 7, 8 oder 9, dadurch gekennzeichnet, daß die Menge in das Drehscheiben-Einkapselgerät zugeführten Materials zwischen 50 g und 200 g pro Minute beträgt.

11. Verfahren nach einem der Ansprüche 7 bis 10, dadurch gekennzeichnet, daß die freie Fettsäure eine C₁₂ bis C₂₄ freie Fettsäure ist.

12. Verfahren nach Anspruch 11, dadurch gekennzeichnet, daß die freie Fettsäure Stearinsäure ist.

13. Verfahren nach einem der Ansprüche 7 bis 12, dadurch gekennzeichnet, daß es sich bei den Bakterien um Enterococcus faceium handelt.

## Revendications

1. Capsules individuelles discrètes de bactéries microencapsulées avec un acide gras, que l'on peut produire en mélangeant une culture bactérienne lyophilisée avec un acide gras et en encapsulant ledit mélange au moyen d'un disque rotatif.

2. Bactéries microencapsulées selon la revendication 1, caractérisées en ce que l'acide gras est un acide gras libre en C₁₂ à C₂₄.

3. Bactéries microencapsulées selon la revendication 2, caractérisées en ce que l'acide gras est l'acide stéarique.

4. Bactéries microencapsulées selon la revendication 1, 2 ou 3, caractérisées en ce que les capsules microencapsulées sont constituées d'un acide gras à 50% à 90%, le reste étant une culture bactérienne.

5. Bactéries microencapsulées selon l'une quelconque des revendications précédentes, caractérisées en ce que les capsules ont une taille particulaire comprise entre 75 micromètres et 300 micromètres.

6. Bactéries microencapsulées selon l'une quelconque des revendications précédentes, caractérisées en ce qu'elles appartiennent à l'espèce Enterococcus faceium.

7. Procédé de traitement de capsules individuelles discrètes de bactéries microencapsulées avec un acide gras, ledit procédé comprenant:
l'obtention d'une culture bactérienne faite de bactéries séchées, le mélange de ladite culture lyophilisée avec de 50% à plus de 90% en poids d'une masse fondue d'acide gras, et
l'encapsulation au moyen d'un disque rotatif du mélange d'acide gras et de bactéries lyophilisées dans un encapsulateur à disque rotatif pour fournir des capsules de bactéries à écoulement libre revêtues individuellement.

8. Procédé selon la revendication 7, caractérisé en ce que l'encapsulateur à disque rotatif opère à une vitesse comprise entre 2000 tpm et 4000 tpm (tours par minute).

9. Procédé selon la revendication 7 ou 8, caractérisé en ce que l'encapsulateur à disque rotatif opère dans un intervalle de vitesses compris entre 2500 tpm et 3200 tpm.

10. Procédé selon la revendication 7, 8 ou 9, caractérisé en ce que la vitesse d'introduction de la matière dans l'encapsulateur à disque rotatif est de 50 grammes par minute à 200 grammes par minute.

11. Procédé selon l'une quelconque des revendications 7 à 10, caractérisé en ce que l'acide gras libre est un acide gras libre en C₁₂ à C₂₄.

12. Procédé selon la revendication 11, caractérisé en ce que l'acide gras libre est l'acide stéarique.

13. Procédé selon l'une quelconque des revendications 7 à 12, caractérisé en ce que les bactéries appartiennent à l'espèce Enterococcus faceium.
